(19) Europäisches Patentamt
European Patent Office
Office européen des brevets

(11) Veröffentlichungsnummer: **0 305 924 B1**

(12) # EUROPÄISCHE PATENTSCHRIFT

(45) Veröffentlichungstag der Patentschrift: **09.10.91 Patentblatt 91/41**

(51) Int. Cl.$^5$: **C07B 45/02**

(21) Anmeldenummer: **88113963.8**

(22) Anmeldetag: **26.08.88**

(54) **Verfahren zur Sulfonierung und/oder Sulfatierung organischer Komponenten mit SO3 in einem organischen Reaktionsmedium.**

(30) Priorität: **03.09.87 DE 3729416**

(43) Veröffentlichungstag der Anmeldung: **08.03.89 Patentblatt 89/10**

(45) Bekanntmachung des Hinweises auf die Patenterteilung: **09.10.91 Patentblatt 91/41**

(84) Benannte Vertragsstaaten: **AT BE CH DE ES FR GB IT LI NL SE**

(56) Entgegenhaltungen: **DE-A- 2 320 933**

(73) Patentinhaber: **Henkel Kommanditgesellschaft auf Aktien
Postfach 1100 Henkelstrasse 67
W-4000 Düsseldorf-Holthausen (DE)**

(72) Erfinder: **Kretschmann, Josef, Dr.
Friedensstrasse 19
W-4018 Langenfeld (DE)**
Erfinder: **Carduck, Franz-Josef, Dr.
Landstrasse 18
W-5657 Haan (DE)**
Erfinder: **Wüst, Willi, Dr.
Fasanenring 32
W-4030 Ratingen (DE)**
Erfinder: **Harth, Huberth, Dr.
Tizian Weg 9
W-4010 Hilden (DE)**
Erfinder: **Springer, Dirk, Dr.
Carl-Barth-Strasse 5
W-5657 Haan (DE)**

EP 0 305 924 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Sulfierung organischer Verbindungen durch deren Umsetzung mit Schwefeltrioxid $SO_3$ erfaßt in der Praxis insbesondere die Reaktionstypen der Sulfonierung und/oder der Sulfatierung, deren Reaktionsschema bekanntlich wie folgt dargestellt werden kann.

$$\text{Sulfonierung : } R-H + SO_3 \rightarrow RSO_3H$$

$$\text{Sulfatierung : } R-OH + SO_3 \rightarrow ROSO_3H$$

Reaktionen dieses Typs zeichnen sich durch eine sehr schnelle Kinetik aus, sie sind aber auch mit der Abgabe einer hohen Reaktionswärme belastet, die beispielsweise 100 bis 180 kJ/mol betragen kann. Bei Reaktionssystemen mit vergleichsweise hoher Viskosität bringt das Probleme bei der Wärmeabführung, so daß in aller Regel lokale Überreaktionen, Überhitzungen, Verkohlungen, Dehydratisierungen und/oder Polymerisationen ausgelöst werden können. Oxidationsreaktionen lösen Verfärbungen aus, durch die die Qualität der Reaktionsprodukte negativ beeinflußt wird, so daß Bleich- und Reinigungsschritte an den Sulfonierungs- und/oder Sulfatierungsprodukten erforderlich sind.

Unter den technischen Ausführungsformen dieser Umsetzungen nimmt eine Gas-Flüssig-Reaktion mit gasförmigem Schwefeltrioxid, verdünnt in einem Inertgas, an dünnen Flüssigkeitsfilmen eine herausragende Stellung ein.

Organische Verbindungen können zwar in vielen Fällen unter milderen Reaktionsbedingungen sulfoniert und/oder sulfatiert werden, wenn an Stelle von Schwefeltrioxid Chlorsulfonsäure, Schwefelsäure oder Oleum eingesetzt wird. Hier macht jedoch der sich entwickelnde Chlorwasserstoff bzw. die entstehende verdünnte Schwefelsäure eine mühevolle Abtrennung derselben erforderlich. Schließlich gelingt die Sulfonierung und/oder Sulfatierung vieler besonders empfindlicher Substrate mit herkömmlichen Mitteln nur sehr unbefriedigend oder überhaupt nicht.

Um diese Nachteile während der Sulfonierung und/oder Sulfatierung zu verringern, wenn nicht sogar auszuschalten, wurde versucht, die extreme Reaktivität von Schwefeltrioxid durch Lösungsmittelzusatz herabzusetzen. Diese Verwendung von Lösungsmitteln ermöglicht eine bessere Verteilung und Durchmischung der Reaktanten und bei problemloser Wärmeabführung eine isotherme Reaktionsführung (siehe beispielsweise : E.E. Gilbert in "Chemical Reviews" 62, 549 bis 580 (1962) ; Kirk-Othmer in "Encyclopedia of Chemical Technology" 22, 1 bis 45 (1983).

Als Lösungsmittel sind beispielsweise vorgeschlagen worden Schwefeldioxid oder halogenierte Kohlenwasserstoffe. Die Verwendung des giftigen und hochkorrosiven flössigen Schwefeldioxids löst Probleme bei seiner Handhabung aus und bleibt überdies nur Reaktionen verhältnismäßig unempfindlicher Aromaten vorbehalten. Auch die weiteren vorgeschlagenen Lösungsmittel für Schwefeltrioxid wie niedrig siedende halogenierte Kohlenwasserstoffe, Paraffine, cyclische Ether sowie tertiäre Amine können beträchtliche Schwierigkeiten auslösen. Lösungsmittel dieser Art können mit $SO_3$ heftig ablaufende Reaktionen ergeben, so daß die Herstellung und Handhabung solcher Lösungen in vielen Fällen mit Schwierigkeiten verbunden ist. Nach beendeter Sulfonierung und/oder Sulfatierung müssen neben dem Lösungsmittel noch Nebenreaktionsprodukte in zumeist recht mühevoller Weise abgetrennt werden, die während der Herstellung der Sulfonierungs- und/oder Sulfatierungsmittel entstanden sind.

In DE-A-2320933 wird ein Verfahren zur Dünnfilmsulfonierung sulfonierbarer Olefine und/oder höherer Alkohole beschrieben, das in Gegenwart von 30 bis 10 000 ppm eines nichtionischen Netzmittels durchgeführt wird.

Die Lehre der Erfindung geht von der Aufgabe aus, für die Sulfierung organischer Komponenten mit $SO_3$ ein sicheres und vergleichsweise unempfindliches, unter den Reaktionsbedingungen in flüssiger Phase vorliegendes Reaktionsmedium zu schaffen, das eine verbesserte Reaktionskontrolle und -steuerung dergestalt ermöglicht, daß unerwünschte Nebenreaktionen wenigstens weitgehend oder auch praktisch vollständig ausgeschaltet werden können. Die Erfindung will damit nicht nur an sich bekannte Sulfierungsreaktionen der angegebenen Art besser kontrollierbar ausgestalten, es soll mit den Arbeitsmitteln der Erfindung auch möglich werden, gezielte Sulfierungsreaktionen an hoch empfindlichen Substraten vorzunehmen, die bisher für eine Sulfierung mit $SO_3$ nicht in Betracht gezogen werden konnten.

Die technische Lösung der erfindungsgemäßen Aufgabenstellung geht von der überraschenden Erkenntnis aus, daß bestimmt ausgewählte Carbonsäureester als den Reaktionsablauf moderierendes Reaktionsmedium für den angegebenen Zweck besonders geeignet sind.

Carbonsäureester sind an sich bekannte aktive Reaktanten für Sulfierungsreaktionen mit $SO_3$. Die Praxis

macht von dieser chemischen Reaktion in großem Umfange Gebrauch bei der Herstellung in alpha-Stellung sulfonierter Fettsäureester bzw. entsprechender alphasulfonierter Fettsäuren und/oder ihrer Salze. Praktische Bedeutung hat diese Reaktion insbesondere bei der Herstellung tensidaktiver Komponenten aus den Klassen der Estersulfonatsalze und/oder die Salze von alpha-Sulfo-Fettsäuren. Charakterisches und übereinstimmendes Element für diese Sulfonierungsreaktion der hier besprochenen Art ist die Einführung der Sulfosäuregruppe in die alpha-Stellung der Carbonsäure.

Von diesem Wissen ausgehend, schlägt die Erfindung vor, als verfahrenssteuerndes Reaktionsmedium ausgewählte Ester solcher Carbonsäuren zu verwenden, die zu einer Sulfonierung in alpha-Position nicht befähigt sind und auch keine sonstigen Strukturelemente enthalten, die Anlaß für unerwünschte Sekundärreaktionen, insbesondere mit dem aggressiven $SO_3$, sein könnten.

Gegenstand der Erfindung ist dementsprechend die Verwendung ausgewählter, unter Anwendungsbedingungen flüssiger Carbonsäureester der allgemeinen Formel I

$$X\text{-}COOR^1 \quad (I)$$

in der $R^1$ einen in alpha-Stellung unverzweigten Alkylrest mit 1-8 C-Atomen und X Wasserstoff oder einen Rest der allgemeinen Formel II

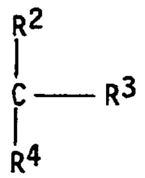

II

bedeuten, worin $R^2$, $R^3$ und $R^4$ für Fluor und/oder Chlor stehen und dabei einer dieser Reste auch Wasserstoff bzw. ein oder zwei dieser Reste auch einen Alkylrest mit bis zu 6 C-Atomen bedeuten können, als Reaktionsmedium für die Sulfonierung und/oder Sulfatierung organischer Komponenten mit $SO_3$ zu hellfarbigen Umsetzungsprodukten.

Weiterer Erfindungsgegenstand ist ein Verfahren zur Gewinnung hellfarbiger Reaktionsprodukte bei der Sulfonierung und/oder Sulfatierung organischer Komponenten mit $SO_3$, welches dadurch gekennzeichnet ist, daß als Reaktionsmedium unter Anwendungsbedingungen flüssige Carbonsäureester der allgemeinen Formel I eingesetzt werden, in der X und $R^1$ die angegebene Bedeutung haben und daß weiterhin nach Abschluß der Reaktion die Carbonsäureester insbesondere durch Destillation und/oder Flüssigextraktion von den Sulfonierungs- bzw. Sulfatierungsprodukten abgetrennt werden.

Die durch die oben angegebene allgemeine Formel I dargestellten und erfindungsgemäß ausgewählten Carbonsäureester zeichnen sich übereinstimmend dadurch aus, daß sie einer alpha-Sulfonierung im Sinne der Herstellung von alpha-Sulfocarbonsäureestern nicht zugänglich sind. Sie sind im allgemeinen gute Lösungsmittel für die zu sulfonierenden bzw. zu sulfatierenden organischen Komponenten — sofern eine Löslichkeit dieser Verbindung überhaupt gegeben ist — was erfindungsgemäß nicht zwingend gefordert wird. Auch die Umsetzung feindisperser Feststoffsuspensionen unlöslicher organischer Reaktanten in den erfindungsgemäß ausgewählten Carbonsäureestern fällt in den Rahmen der Erfindung.

Dieses Reaktionsmedium auf Esterbasis besitzt weiterhin die Möglichkeit, $SO_3$ molekulardispers gelöst aufzunehmen und in dieser Form der gesteuerten Umsetzung zuzuführen.

Insbesondere zur erleichterten Abtrennung des als Reaktionsmedium eingesetzten erfindungsgemäßen Hilfsmittels kann es bevorzugt sein, Carbonsäureester der genannten Art unter Berücksichtigung ihrer Siedecharakteristiken auszuwählen. Bevorzugte Reaktionsmedien der allgemeinen Formel I sind Ester mit Siedepunkten bei Normaldruck bis in den Bereich von etwa 250°C vorzugsweise bis in den Bereich von etwa 170°C. Die destillative Abtrennung solcher Carbonsäureester der allgemeinen Formel I ist nach Abschluß des Sulfierverfahrens — gewünschtenfalls im Vakuum — leicht möglich.

Die Siedecharakteristik des für den jeweiligen Fall auszuwählenden Carbonsäureesters kann aber auch eine darüber hinausgehende Bedeutung haben. So kann insbesondere die maximale Innentemperatur des Reaktionsgemisches durch die Siedetemperatur des erfindungsgemäßen Reaktionsmediums unter den Verfahrensbedingungen vorher bestimmt werden. Durch Auswahl der geeigneten esterbildenden Säurekomponente einerseits und der zugehörigen Alkoholkomponente andererseits kann die Siedecharakteristik des Esters und damit die gewünschte Maximaltemperatur unter den Umsetzungsbedingungen vorher bestimmt werden. Überschießende Reaktionswärmebeträge aus der in diesem Reaktionsmedium stattfindenden Umsetzung — Sulfonierung und/oder Sulfatierung — führen unmittelbar zur anteilsweisen Verdampfung des Reaktionsmediums und damit zur sicheren Temperatursteuerung unter Wahrung vorgegebener Maximaltemperaturen. Erstaunlicherweise erschließt sich hier eine besonders einfache Möglichkeit zur Herstellung hellfarbiger und/oder in anderer Weise temperatursensibler Reaktionsprodukte der angestrebten Art.

Der Rest X in den ausgewählten Carbonsäureestern der allgemeinen Formel I kann Wasserstoff bedeuten. Betroffen sind damit generell Ameisensäureester der genannten Art. Ein alpha-Kohlenstoffatom liegt in dem Carbonsäurerest nicht vor, so daß diese Reaktionsmöglichkeit ausscheidet. Auf der Alkoholseite des Esters ist lediglich auszuschließen, daß unerwünschte Esterzersetzungen unter der Einwirkung von $SO_3$ stattfinden. Die erfindungsgemäße Lehre fordert deswegen für den Alkoholrest $R^1$ den Ausschluß einer Verzweigung in alpha-Stellung. Im übrigen können diese Alkylreste geradkettig oder verzweigt sein. Die Kettenlänge dieses Alkoholrestes bestimmt den Siedepunkt des Esters. Hier sind die zuvorgenannten bevorzugten oberen Siedegrenzen für das erfindungsgemäße Reaktionsmedium zu berücksichtigen. $R^1$ stellt einen Alkylrest mit 1-8 C-Atomen, insbesondere mit nicht mehr als 6 C-Atomen dar. Die Methyl-, Ethyl-, Propyl- und/oder Butylester besitzen im allgemeinen besondere Bedeutung. Das gilt nicht nur für die hier besprochenen Ameisensäureester, sondern auch für die andere Gruppe der erfindungsgemäß zum Einsatz kommenden Reaktionshilfsmittel, in denen X einen Rest der allgemeinen Formel II darstellt.

Ester dieser zuletzt genannten Art leiten sich von verschiedenartigsten halogensubstituierten Carbonsäuren ab, die sich allerdings durchgängig dadurch auszeichnen, daß sie unter den erfindungsgemäß eingesetzten Reaktionsbedingungen mit $SO_3$ zu keiner alpha-Sulfonierung führen. Der Alkoholanteil der Ester aus dieser zweiten Gruppe ist mit den im Zusammenhang mit den Ameisensäureestern beschriebenen Alkohol übereinstimmend. Die Auswahl niederer Alkohole der angegebenen Art, insbesondere Methylund/oder Ethylalkohol — fördert die destillative Abtrennbarkeit der Reaktionshilfsmittel nach Abschluß der gewünschten Sulfierung.

Als Säurekomponente in den hier betroffenen Reaktionsmedien sind zunächst die Trifluor- und die Trichloressigsäure zu nennen. Trifluoressigsäureester besitzen besonders niedrige Siedepunkte, die Trichloressigsäure ist eine besonders wohlfeile Chemikalie, so daß die Verwendung ihrer Ester aus wirtschaftlichen Gesichtspunkten interessant sein kann.

Einer der Reste $R^2$, $R^3$ bzw. $R^4$ aus der allgemeinen Formel II kann Wasserstoff bedeuten. Die zwei am gleichen Kohlenstoff vorhandenen Halogenatome schließen die alpha-Sulfonierung als unerwünschte Nebenreaktion mit hinreichender Sicherheit aus. Einer oder zwei dieser Reste $R^2$, $R^3$ und $R^4$ können aber auch Niedrigalkylreste sein. Als Niedrigalkylreste sind hier solche mit bis zu 6, insbesondere mit bis zu 3 C-Atomen zu verstehen. Auch in diesem Fall ist hinreichende Beständigkeit gegen unerwünschten $SO_3$-Angriff im alpha-Kohlenstoffatom sichergestellt.

Die Durchführung der erfindungsgemäß unter Mitverwendung ausgewählter Carbonsäureester moderierten Sulfonierungs- und/oder Sulfatierungsreaktionen kann in vielgestaltiger Form vorgenommen bzw. variiert werden. So ist es insbesondere möglich, daß $SO_3$ und/oder die umzusetzenden organischen Komponenten wenigstens anteilsweise vorgemischt mit Carbonsäureestern der allgemeinen Formel I in der Reaktionsstufe zum Einsatz zu bringen. In einer Ausführungsform werden dabei Lösungen der Reaktanten in den Carbonsäureestern der allgemeinen Formel I der Reaktionsstufe zugeführt und hier insbesondere unter Temperaturkontrolle miteinander vermischt. Es kann dabei sowohl die organische umzusetzende Komponente als auch das $SO_3$ bzw. die $SO_3$ liefernde Komponente mit den erfindungsgemäß eingesetzten Reaktionshilfsmittel vermischt zur Anwendung kommen.

Bei der Durchführung eines solchen Verfahrens, das insbesondere für die absatzweise Reaktionsführung geeignet ist, wird es im allgemeinen bevorzugt sein, die zu sulfierende organische Komponente zusammen mit einem Anteil des erfindungsgemäß als Reaktionshilfsmittel eingesetzten Carbonsäureesters vorzulegen und $SO_3$ dann — vorzugsweise ebenfalls in solchen erfindungsgemäßen Reaktionshilfsmitteln gelöst — der Reaktionszone in dem Ausmaße zuzugeben, in dem die dabei entstehende Temperaturerhöhung aufgefangen und gewünschtenfalls abgeführt werden kann.

In einer anderen Ausführungsform des erfindungsgemäßen Verfahrens kann aber auch die an sich bekannte insbesondere kontinuierlich geführte Mischphasenreaktion verbessert werden, bei der die zu sulfierende organische Komponente als Flüssigphase in dünner Schicht einer $SO_3$ enthaltenden Gasphase ausgesetzt wird. Hier ist es erfindungsgemäß insbesondere möglich, den Reaktionsablauf dadurch zu moderieren, daß als Flüssigphase eine Lösung der zu sulfierenden organischen Komponenten vorgegeben wird, die in dünner Schicht mit einem $SO_3$ enthaltenden Gasstrom im Gleichstrom oder im Gegenstrom in Kontakt gebracht wird. Als Gasphase kann $SO_3$ als solches, insbesondere aber auch $SO_3$ vermischt mit Inertgas, wie Stickstoff oder vermischt mit Luft zum Einsatz kommen.

Die in alpha-Stellung nicht sulfonierbaren Carbonsäureester können mit Schwefeltrioxid und/oder den zu sulfierenden organischen Komponenten in jedem Verhältnis gemischt werden. Mit Schwefeltrioxid bilden sie homogene Lösungen. Der $SO_3$-Gehalt der erfindungsgemäß eingesetzten Lösungen hängt von den Eigenschaften der zu sulfonierenden und/oder sulfatierenden organischen Verbindungen ab. Je empfindlicher diese

organischen Substrate beispielsweise gegenüber Oxidationen sind, umso geringer kann der $SO_3$-Gehalt der zum Einsatz kommenden Lösungen gewählt werden. Üblicherweise werden jedoch Lösungen mit $SO_3$-Gehalten zwischen 10 und 60 Gewichtsprozent, insbesondere zwischen 20 und 40 Gewichtsprozent, eingesetzt. In manchen Fällen kann die Verwendung von niedrigeren oder auch von höheren $SO_3$-Gehalten vorteilhaft bzw. möglich sein.

Die Temperatur der Reaktionszone überschreitet im allgemeinen etwa 100°C nicht. Vorteilhafterweise wird bei Temperaturen von höchstens etwa 80°C gearbeitet. Besonders geeignete Reaktionstemperaturen liegen im Bereich bis etwa 60°C beispielsweise von etwa 0 bis 40°C. Die Auswahl geeigneter Ester der erfindungsgemäßen Definition macht gewünschtenfalls auch das Arbeiten unterhalb 0°C möglich, beispielsweise im Temperaturbereich von minus 25 bis plus 20°C. Es leuchtet ein, daß hier besonders sichere Verfahrenssteuerungen möglich werden, die den einfachen Zugriff auf bisher nicht für möglich gehaltene Sulfonierungen und/oder Sulfatierungen ergeben.

Die gewünschten Reaktionstemperaturen können durch Abführung der Reaktionswärme mittels Kühlung oder — wie bereits angegeben — durch anteilsweise Verdampfung des erfindungsgemäßen Reaktionshilfsmittels eingehalten bzw. eingestellt werden. Ein besonders einfacher Indikator für unerwünschte Nebenreaktionen ist die Farbe des Reaktionsgemisches. Sie gibt im allgemeinen Aufschluß über das Ausmaß unerwünschter Nebenreaktionen. Erfindungsgemäß können selbst heikelste Umsetzungen zu farblosen oder höchstens sehr schwach eingefärbten Reaktionsprodukten eingesetzt werden, deren nachfolgende Reinigung keine Schwierigkeiten bereitet, sofern sie gewünscht wird.

Die Entfernung des erfindungsgemäßen Reaktionshilfsmittels auf Carbonsäureester-Basis kann nicht nur durch Destillation, sondern häufig auch durch selektive Extraktion erfolgen. Die Carbonsäureester der definierten Art sind vergleichsweise hydrophobe Bestandteile, während sich die als Reaktionsprodukt gewünschten Sulfonierungs- und/oder Sulfatierungsprodukte im allgemeinen durch einen ausgeprägt hydrophilen Charakter auszeichnen. Diese Unterschiede können in an sich bekannter Weise zur extraktiven Trennung benutzt werden. Dabei kann beispielsweise durch Salzbildung an den Sulfierungsprodukten — etwa durch Bildung der entsprechenden Natriumsalze — zusätzlich Einfluß auf die verbesserte Trennung zwischen Reaktionsprodukt und Reaktionshilfsmittel im Sinne der Erfindung genommen werden.

Das erfindungsgemäße Verfahren kann im breiten Rahmen der bekannten Sulfonierung und/oder Sulfatierung organischer Komponenten zum Einsatz kommen und wird insbesondere überall dort besonders vorteilhaft Verwendung finden, wo auf definierte Umsetzungen unter weitgehendem Ausschluß unerwünschte Nebenreaktionen Wert zu legen ist.

Das Arbeiten mit den erfindungsgemäßen Hilfsmitteln erschließt aber darüber hinaus die Möglichkeit, an sich labile organische Komponenten einer gezielten Sulfonierung und/oder Sulfatierung zu unterwerfen, die mit den bisher zur Verfügung stehenden Arbeitsmitteln nicht durchzuführen waren. Beispiele hierfür sind :

## Beispiel 1

Es wurden 2,98 g (27,3 mmol) Schwefeltrioxid in 7,5 g Trifluoressigsäuremethylester gelöst und unter Rühren zu einer Lösung von 5,70 g (36,0 mmol) Decanol-1 in 17,1 g Trifluoressigsäuremethylester getropft, wobei die Temperatur durch Verdampfung des Lösungsmittels bei etwa 30°C gehalten wurde. Anschließend wurde auf dem Wasserbad bei 55°C das Lösungsmittel abdestilliert und der Rückstand mit Natronlauge neutralisiert.

Die Ausbeute an Natriumdecylsulfat wurde nach DIN/ISO 2271 bestimmt und betrug 9,36 g (100%).

## Beispiel 2

Es wurden 65,2 g (0,82 mol) Schwefeltrioxid in 175 g Trichloressigsäuremethylester gelöst und unter Rühren auf dem Eisbad zu einer Lösung von 122,6 g Decanol-1 (0,77 mol) in 368 g Trichloressigsäuremethylester getropft, wobei die Temperatur unter 15°C gehalten wurde. Trichloressigsäuremethylester wurde bei 70°C/0,1 Torr abdestilliert und der Rückstand mit Natronlauge neutralisiert.

Die Ausbeute an Natriumdecylsulfat wurde nach DIN/ISO 2271 bestimmt und betrug 187,2 g (93%).

## Beispiel 3

Es wurden 19,2 g (0,24 mol) Schwefeltrioxid in 80 g Dichloressigsäuremethylester gelöst und unter Rühren auf dem Eisbad zu einer Lösung von 37,2 g (0,20 mol) Dodecanol-1 in 10 g Dichloressigsäuremethylester getropft, wobei die Temperatur auf 30°C anstieg. Anschließend wurde das Lösungsmittel abdestilliert und der Rückstand mit Natronlauge neutralisiert.

Die Ausbeute an Natriumdodecylsulfat wurde nach DIN/ISO 2271 bestimmt und betrug 43 g (80%).

Beispiel 4

In 10 g Trifluoressigsäureethylester wurden 9,0 g (0,11 mol) Schwefeltrioxid gelöst und unter Rühren so schnell zu einer Lösung von 12,0 g (0,11 mol) m-Xylol in 5 g Trifluoressigsäureethylester getopft, daß die Temperatur 20°C nicht überstieg. Nach beendeter Zugabe wurde eine 1/4 Stunde unter Rückfluß erhitzt. Beim Abkühlen kristallisierte 3,5-Dimethylbenzolsulfonsäure aus. Es wurden 12,7 g (54%) farblose, hygroskopische Blättchen erhalten, die an der Luft rasch zerfließen. Durch Einengen der Mutterlauge wurden weitere 4,6 g (20%) Produkt gewonnen. Zur Charakterisierung wurden 2 g der Sulfonsäure in das entsprechende Säureamid überführt und ein Schmelzpunkt von 137°C ermittelt (Literatur : 137°C).

Beispiel 5

Es wurden 8,7 g (0,11 mol) Schwefeltrioxid in 18,8 g Trichloressigsäuremethylester gelöst und unter Rühren so schnell zu einer Lösung von 26,1 g (0,11 mol) technischem Alkylbenzol (Molekulargewicht = 240) in 60,9 g Trichloressigsäuremethylester getropft, daß durch Außenkühlung die Temperatur 20°C nicht überstieg. Anschließend wurde das Lösungsmittel im Vakuum abdestilliert und der Rückstand mit Natronlauge neutralisiert.

Die Ausbeute an Natriumalkylbenzolsulfonat wurde nach DIN/ISO 2271 bestimmt und betrug 35,5 g (96%).

Beisiel 6

In 40 g Butylformiat wurden 5 g (0,06 mol) Schwefeltrioxid gelöst und bei 80°C zu einer Lösung von 80 g technischem $C_{10-14}$-Fettalkoholglucosid in 20 g Butylformiat getropft. Nach 10 Minuten wurde die sich wachsartig verfestigende Masse unter vermindertem Druck vom Lösungsmittel befreit und mit Natronlauge neutralisiert. Das Reaktionsprodukt wurde im Hochvakuum getrocknet und in einer Labormühle gemahlen. Es wurden 84 g eines hellgelben, wasserlöslichen Produktes erhalten, welches 2,3% organisch gebundenen Schwefel enthielt.

Beispiel 7

Ein Gemisch aus 22 g (0,28 mol) Schwefeltrioxid und 150 g Ameisensäureethylester wurde bei etwa 22°C tropfenweise zu 263 g (0,28 mol) Rizinusöl gegeben und 12 Stunden bei etwa 22°C belassen. Anschließend wurde das Lösungsmittel abdestilliert und der Rückstand mit Natronlauge neutralisiert. Der Gehalt an $SO_3Na$ wurde nach DIN/ISO 2271 bestimmt und betrug 5 Gew.-% $SO_3$.

Beispiel 8

Es wurden 1,5 g (19 mmol) Schwefeltrioxid in 5 g Trichloressigsäuremethylester gelöst und unter Rühren zu einer Lösung von 20 g (21 mmol) Rizinusöl in 40 g Trichloressigsäuremethylester getropft, wobei durch Außenkühlung mit Hilfe eines Eisbades die Temperatur unter 20°C gehalten wurde. Das Lösungsmittel wurde bei 60°C/0,1 Torr abdestilliert und der Rückstand mit Natronlauge neutralisiert. Der $SO_3Na$-Gehalt wurde nach DIN/ISO 2271 bestimmt und betrug 6,3 Gew.% $SO_3$.

## Patentansprüche

1. Verwendung ausgewählter, unter Anwendungsbedingungen flüssiger Carbonsäureester der allgemeinen Formel I

$$X\text{-}COOR^1$$

in der $R^1$ einen in alpha-Stellung unverzweigten Alkylrest mit 1-8 C-Atomen und X Wasserstoff oder einen Rest der allgemeinen Formel II

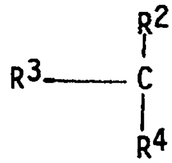

bedeuten, worin $R^2$, $R^3$ und $R^4$ für Fluor und/oder Chlor stehen und dabei einer dieser Reste auch Wasserstoff bzw. ein oder zwei dieser Reste auch einen Alkylrest mit bis zu 6 C-Atomen bedeuten können, als Reaktionsmedium für die Sulfonierung und/oder Sulfatierung organischer Komponenten mit $SO_3$ zu hellfarbigen Umsetzungsprodukten.

2. Verwendung nach Anspruch 1, dadurch gekennzeichnet, daß das $SO_3$ und/oder die umzusetzenden organischen Komponenten wenigstens anteilsweise vorgemischt mit Carbonsäureestern der allgemeinen Formel I in der Reaktionsstufe zum Einsatz kommen.

3. Verwendung nach Ansprüchen 1 und 2, dadurch gekennzeichnet, daß Lösungen der Reaktanten in den Carbonsäureestern der allgemeinen Formel I der Reaktionsstufe zugeführt und hier insbesondere unter Temperaturkontrolle miteinander vermischt werden, wobei bevorzugt Reaktionstemperaturen von 80°C nicht überschritten werden.

4. Verwendung nach Ansprüchen 1-3, dadurch gekennzeichnet, daß gasförmiges $SO_3$—gewünschtenfalls in Abmischung mit einem Inertgas — der Umsetzungsstufe zugeführt wird, in der die zu sulfonierenden und/oder zu sulfatierenden Komponenten gelöst und/oder suspendiert in den Carbonsäureestern der allgemeinen Formel I vorliegen.

5. Verwendung nach Ansprüchen 1-4, dadurch gekennzeichnet, daß Carbonsäureester der allgemeinen Formel I mit Siedepunkten bei Normaldruck nicht oberhalb 250°C, vorzugsweise nicht oberhalb 170°C verwendet werden.

6. Verfahren zur Gewinnung hellfarbiger Reaktionsprodukte bei der Sulfonierung und/oder Sulfatierung organischer Komponenten mit $SO_3$, dadurch gekennzeichnet, daß als Reaktionsmedium unter Anwendungsbedingungen flüssige Carbonsäureester der allgemeinen Formel I

$$X\text{-}COOR^1$$

eingesetzt werden, in der $R^1$ und X die in Anspruch 1 angegebene Bedeutung haben und daß nach Abschluß der Reaktion der Carbonsäureester insbesondere durch Destillation und/oder Flüssigextraktion von den Sulfonierungs- bzw. Sulfatierungsprodukten abgetrennt werden.

7. Verfahren nach Anspruch 6, dadurch gekennzeichnet, daß Reaktionstemperaturen von etwa 40°C nicht überschritten werden, wobei die Abführung der Reaktionswärme durch Kühlung, gewünschtenfalls auch durch anteilsweise Verdampfung des Reaktionsmediums, erfolgen kann.

8. Verfahren nach Ansprüchen 6 und 7, dadurch gekennzeichnet, daß die Umsetzung in an sich bekannten Dünnschichtreaktoren erfolgt, wobei eine $SO_3$-haltige Gasphase im Gleich- oder Gegenstrom zu einer Flüssigphase geführt wird, die die umzusetzenden organischen Komponenten gelöst in Carbonsäureestern der allgemeinen Formel I enthält.

## Claims

1. The use of selected carboxylic acid esters which are liquid under in-use conditions and which correspond to the following general formula

$$X\text{-}COOR^1 \quad (I)$$

in which $R^1$ is a $C_{1-8}$ alkyl radical unbranched in the α-position and X is hydrogen or a group corresponding to the following general formula

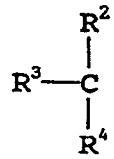

(II)

in which $R^2$, $R^3$ and $R^4$ represent flourine and/or chlorine and one of these substituents may also be hydrogen or one or two of these substituents may also be an alkyl radical containing up to 6 carbon atoms, as reaction medium for the sulfonation and/or sulfatization of organic components with $SO_3$ to light-coloured reaction products.

2. The use claimed in claim 1, characterized in that the $SO_3$ and/or the organic components to be reacted are used in the reaction stage in at least partly premixed form with carboxylic acid esters corresponding to general formula I.

3. The use claimed in claims 1 and 2, characterized in that solutions of the reactants in the carboxylic acid esters corresponding to general formula I are delivered to the reaction stage where they are mixed with one another, in particular under temperature control, reaction temperatures of 80°C preferably not being exceeded.

4. The use claimed in claims 1 to 3, characterized in that gaseous $SO_3$, if desired in admixture with an inert gas, is delivered to the reaction stage in which the components to be sulfonated and/or sulfatized are present in solution and/or suspension in the carboxylic acid esters corresponding to general formula I.

5. The use claimed in claims 1 to 4, characterized in that carboxylic acid esters of general formula I which have boiling points at normal pressure of no more than 250°C and preferably no more than 170°C are used.

6. A process for the production of light-colored reaction products in the sulfonation and/or sulfatization of organic components with $SO_3$, characterized in that carboxylic acid esters liquid under in-use conditions and corresponding to the following general formula

$$X\text{-}COOR^1 \quad (I)$$

in which $R^1$ and X are as defined in claim 1, are used as the reaction medium and in that, on completion of the reaction, the carboxylic acid esters are separated off from the sulfonation or sulfatization products, more especially by distillation and/or liquid extraction.

7. A process as claimed in claim 6, characterized in that reaction temperatures of around 40°C are not exceeded, the heat of reaction being dissipated by cooling and, if desired, even by partial evaporation of the reaction medium.

8. A process as claimed in claims 6 and 7, characterized in that the reaction takes place in thin-layer reactors known per se through which an $SO_3$-containing gas phase is passed in countercurrent to or co-current with a liquid phase containing the organic components to be reacted in solution in carboxylic acid esters corresponding to general formula I.

## Revendications

1. Mise en oeuvre d'esters d'acide carboxylique sélectionnées, à l'état liquide dans les conditions d'application, de la formule générale I

$$X\text{-}COOR^1$$

dans laquelle $R^1$ représente un radical alkyle non ramifié en position alpha, comportant 1 à 8 atomes de C, et où X correspond à l'hydrogène ou à un groupement de la formule générale II

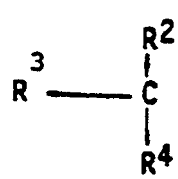

dans laquelle $R^2$, $R^3$ et $R^4$ représentent le fluor et/ou le chlore, tandis que l'un de ces radicaux peut également

correspondre à l'hydrogène, ou un ou deux de ces groupements peuvent également être un radical alkyle comportant jusqu'à 6 atomes de C, comme milieu réactionnel pour la sulfonation et/ou la sulfatation avec $SO_3$ de composés organiques en produits de réaction de couleur claire.

2. Mise en oeuvre selon la revendication 1, caractérisée en ce que le $SO_3$ et/ou les composés organiques à faire entrer en réaction participent à l'étape de réaction, en étant au moins en partie prémélangés avec des esters d'acide carboxylique de la formule générale I.

3. Mise en oeuvre selon les revendications 1 et 2, caractérisée en ce que des solutions des réactifs dans les esters d'acide carboxylique de la formule générale I sont amenées à l'étape de réaction et mélangées entre elles, en particulier, sous contrôle de la température, sans dépasser les températures de réaction préférées de 80°C.

4. Mise en oeuvre selon les revendications 1 à 3, caractérisée en ce que le $SO_3$ gazeux — éventuellement en mélange avec un gaz inerte — est amené à l'étape de réaction dans laquelle les composés à sulfoner et/ou à sulfater sont présents en solution et/ou en suspension dans les esters d'acide carboxylique de la formule générale I.

5. Mise en oeuvre selon les revendications 1 à 4, caractérisée en ce que l'on utilise des esters d'acide carboxylique de la formule générale I dont le point d'ébullition à la pression normale ne dépasse pas 250°C, de préférence n'excède pas 170°C.

6. Procédé de préparation de produits réactionnels de couleur claire par sulfonation et/ou sulfatation de composés organiques avec $SO_3$, caractérisé en ce que l'on utilise comme milieu réactionnel des esters d'acide carboxylique, liquides dans les conditions d'application, de la formule générale I

$$X\text{-}COOR^1$$

dans laquelle $R^1$ et X possèdent la signification indiquée dans la revendication 1, et en ce qu'après l'achèvement de la réaction, les esters d'acide carboxylique sont séparés, en particulier par distillation et/ou extraction liquide des produits de sulfonation et/ou de sulfatation.

7. Procédé selon la revendication 6, caractérisé en ce que l'on ne dépasse pas des températures réactionnelles d'environ 40°C, l'évacuation de la chaleur réactionnelle pouvant se dérouler par refroidissement, le cas échéant également par évaporation partielle du milieu réactionnel.

8. Procédé selon les revendications 6 et 7, caractérisé en ce que la réaction se déroule dans des réacteurs à couche mince connus en soi, dans lesquels une phase gazeuse renfermant du $SO_3$ est amenée dans le même sens ou à contre-courant à une phase liquide contenant les composés organiques à faire réagir, dissous dans des esters d'acide carboxylique de la formule générale I.